# EUROPEAN PATENT APPLICATION

(11) **EP 4 379 717 A1**
(43) Date of publication of application: **05.06.2024**
(21) Application number: 22211008.2
(22) Date of filing: 02.12.2022
(51) Int. Cl.: G10L 25/66, G16H 20/70

(54) **A METHOD FOR ASSESSING HORMONE LEVEL IMPACT OF SPEECH**

(71) Applicant: MySpeaker Oy, 02200 Espoo (FI)
(72) Inventor: Chaker, André Noël, 02200 Espoo (FI); Nadbornik, Galith, 02200 Espoo (FI); Buyle, Christophe, 02200 Espoo (FI); Otterström, Mojgan, 02200 Espoo (FI)
(74) Representative: Kolster Oy Ab

(57) **Abstract**

A computer-implemented method is disclosed. The method comprises receiving a speech from a user, detecting at least one of a plurality of speech features from the speech, determining at least one parameter value of a plurality of technical parameters using the at least one speech feature detected, inputting the plurality of technical parameters determined to a machine learning model trained to map the plurality of technical parameters to at least one hormone level impact label, obtaining at least one predicted hormone level impact label of the speech from the machine learning model, and determining information related to the at least one predicted hormone level impact label obtained to be displayed to the user.

## Description

### TECHNICAL FIELD

Various embodiments relate to achieving optimal hormone levels with speech stimulus.

### BACKGROUND

Verbal communication, such as giving a speech, may have an impact on human hormone levels. Information on how to achieve a desired impact on audience hormone levels may be utilized in training speaking skills.

### BRIEF DESCRIPTION

According to an aspect, there is provided the subject matter of the independent claims. Embodiments are defined in the dependent claims.

Some embodiments provide a method for receiving a speech from a user, detecting features from the speech, determining technical parameters values, inputting the technical parameter values to a machine learning model trained to map them to at least one hormone level impact label, obtaining at least one predicted hormone level impact label of the speech from the machine learning model, and determining information related to the at least one predicted hormone level impact label obtained to be displayed to the user.

### BRIEF DESCRIPTION OF THE DRAWINGS

In the following, various example embodiments will be described in greater detail with reference to the accompanying drawings, in which
Figure 1 shows a simplified architecture of a system;
Figures 2 to 5 are flow charts illustrating example functionalities; and
Figure 6 illustrates an exemplary embodiment of an apparatus.

### DETAILED DESCRIPTION OF SOME EMBODIMENTS

The following embodiments are exemplary. Although the specification may refer to "an", "one", or "some" embodiment(s) in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments/examples to consist of only those features that have been mentioned and such embodiments may contain also features/structures that have not been specifically mentioned.
Embodiments and examples of the method described herein may be implemented in any speech training service measuring and analysing a user's speech and giving feedback. Embodiments and examples may be implemented as a speech training service or as an additional service for existing speech training applications.

Different embodiments and examples are described below using single units, models, equipment, and memory, without restricting the embodiments/examples to such a solution. Concepts called cloud computing and/or virtualization may be used. The virtualization may allow a single physical computing device to host one or more instances of virtual machines that appear and operate as independent computing devices, so that a single physical computing device can create, maintain, delete, or otherwise manage virtual machines in a dynamic manner. It is also possible that device operations will be distributed among a plurality of servers, nodes, devices, or hosts. In cloud computing network devices, computing devices, and/or storage devices provide shared resources. Some other technology advancements, such as Software-Defined Networking (SDN), may cause one or more of the functionalities described below to be migrated to any corresponding abstraction or apparatus or device. Correspondingly, Web 3.0, also known as the third-generation internet, implementing for example blockchain technology, may cause one or more of the functionalities described below to be distributed across a plurality of apparatuses or devices. Therefore, all words and expressions should be interpreted broadly, and they are intended to illustrate, not to restrict, the embodiment.

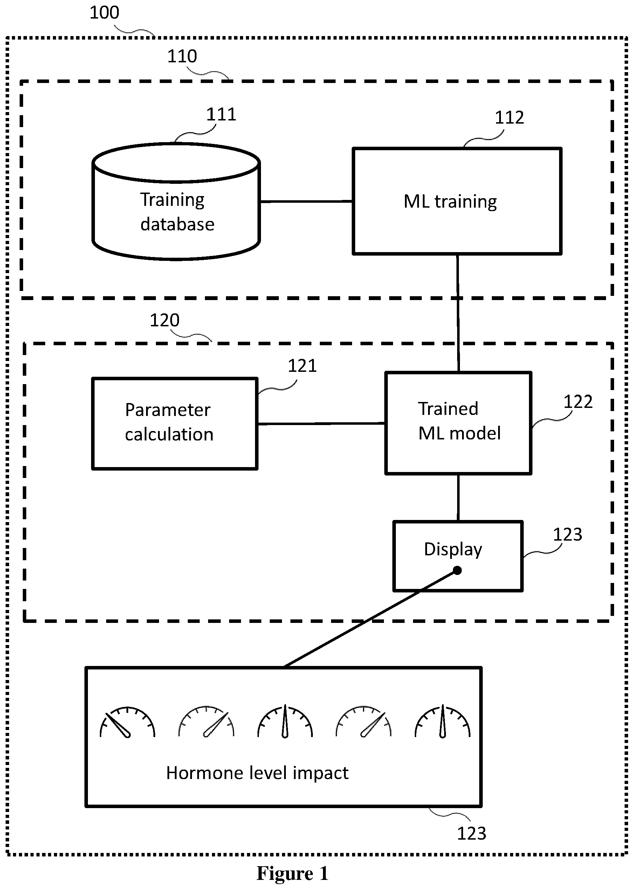

A general exemplary architecture of a system training a machine learning (ML) model and using the trained machine learning model to assess hormone level impact of a speech is illustrated in Figure 1. Figure 1 is a simplified system architecture showing only some devices, apparatuses, and functional entities, all being logical units whose implementation and/or number may differ from what is shown. The connections shown in Figure 1 are logical connections; the actual physical connections may be different. It is apparent to a person skilled in the art that the system comprises any number of shown elements, other equipment, other functions, and other structures that are not illustrated. They, as well as the protocols used, are well known by persons skilled in the art and are irrelevant to the actual invention. Therefore, they need not to be discussed in more detail here.

In the example illustrated by Figure 1, the system 100 comprises equipment for training a machine learning model 110 and equipment for assessing hormone level impact of a speech 120.

Referring to Figure 1, the training equipment 110 for training the machine learning model may be a computing device, such as a personal computer, a laptop, a cloud server, or a backend equipment. The training equipment 110 is configured to comprise a training database 111 comprising training data for training the machine learning model, and a machine learning model trainer unit 112 configured to at least train the machine learning model using data in the training database 111. For that purpose, the equipment comprises a memory and a processor coupled to the memory. The memory may be any kind of conventional or future data repository, including distributed and centralized storing of data, managed by any suitable management system forming part of the training equipment. An example of distributed storing includes a cloud-based storage in a cloud environment (which may be a public cloud, a community cloud, a private cloud, or a hybrid cloud, for example). Cloud storage services may be accessed through a co-located cloud computer service, a web service application programming interface (API) or by applications that utilize API, such as cloud desktop storage, a cloud storage gateway or Web-based content management systems. Further, the training equipment 110 may comprise several servers with databases, which may be integrated to be visible as one database and one database server. However, the manner how training data are stored and retrieved, and the location where different pieces of data to be obtained for the training data are stored are irrelevant to the invention. The processor may comprise one or more processing cores containing circuitry configured to execute instructions. The memory stores processor executable instructions to be executed by the processor to process the training data from the database 111 and training the machine learning model in the training unit 112 as will be described in detail below. The training data may be encoded in the training equipment 110 to a format suitable for the training unit 112, the backend equipment may obtain the data from another device or server in a format suitable for the training unit 112, or the data may be encoded to a suitable format in the training unit 112.

The training data in the training database 111 comprises technical parameters determined using a plurality of speech features detected from a plurality of speeches. The plurality of speech features may be detected from one speech of the plurality of speeches at a time. Technical parameters determined from the speech features are explained in more detail below with a parameter calculation unit 121.

Additionally, the training data for training the machine learning model comprises hormone level impact labels determined using a plurality of hormone levels of different hormones measured from a plurality of individuals while being presented the plurality of speeches. The plurality of hormone levels may be measured from the plurality of individuals while being presented one speech of the plurality of speeches at a time. The different hormones may comprise, for example, cortisol, dopamine, oxytocin, serotonin, and/or dehydroepiandrosterone (DHEA). The plurality of hormone levels may be measured from, for example, blood samples, saliva samples, and/or urine samples of the plurality of individuals taken, for example, before, after, and/or while being presented the plurality of speeches. The hormone level impact labels may comprise information of an increased hormone level, a decreased hormone level, or an unaltered hormone level. The determined hormone level impact labels may be used as a target output for training the machine learning model.

The machine learning model trainer unit 112 configured to at least train the machine learning model using data in the training database 111. The trainer unit 112 may use any machine learning model suitable for supervised learning, that is, algorithms that may be trained on input data labelled for a desired output. The training unit 112 may train the model until the algorithm can detect underlying patterns and relationships between the input data and the output labels, enabling it to yield accurate labelling results when presented with new, unlabelled data. The training unit 112 may use, for example, linear regression as the machine learning model algorithm. The training unit 112 may evaluate the trained machine learning model by, for example, k-fold cross-validation, where a limited sample of the training data is not used for training the machine learning model but for evaluating the quality of the trained machine learning model.

Referring to Figure 1, the assessing equipment 120 refers to a computing device (equipment, apparatus) that may be a portable device or a desktop device such as a personal computer, and it may also be referred to as a user device, a user terminal, or a user apparatus. Portable computing devices (apparatuses) include wireless mobile communication devices operating with or without a subscriber identification module (SIM) in hardware or in software, including, but not limited to, the following types of devices: mobile phone, smartphone, personal digital assistant (PDA), handset, laptop and/or touch screen computer, tablet (tablet computer), multimedia device, wearable computer, and other types of wearable devices, such as clothing and accessories incorporating computer and advanced electronic technologies, augmented reality, mixed reality, or virtual reality devices such as headset displays and sensors. The assessing equipment 120 may comprise one or more user interfaces. The one or more user interfaces may be any kind of a user interface, for example a screen, a keypad, a loudspeaker, a microphone, a camera, a touch user interface, a sensor, an integrated display device, and/or an external display device.

The assessing equipment 120 is configured to support determining and storing technical speech parameters with a parameter calculation unit 121. The assessing equipment 120 is also configured to use the trained machine learning model unit 122 by inputting the technical speech parameters and showing the output on a display 123. For that purpose, the assessing equipment 120 may be capable of installing applications. The assessing equipment 120 may comprise technical parameters and machine learning model output data, permanently or temporarily.

The parameter calculation unit 122 may determine from detected speech features various technical parameters such as, for example, perceived volume, volume variability, pitch, pitch variability, fluidity, pace, pace variability, pauses, facial expressions from neutral, facial expressions variability, filler words, word complexity, openness, extraversion, emotional stability, agreeableness, and/or conscientiousness. The speech features may be detected by the assessing equipment 120 from a live speech provided by a user of the assessing equipment via an interface of the assessing equipment 120, such as a microphone and/or a camera, or the speech features maybe detected from a recording of a speech stored in the assessing unit 120. The speech features may comprise, for example, volume, noise volume, frequency, pauses, words, and/or facial expressions. Perceived volume may be determined by detecting a signal volume and a noise volume during a speech, determining a signal to noise volume, and calculating a mean of the signal to noise volume. The unit used for perceived volume may be decibel (dB). Volume variability may be determined by calculating a standard deviation of the signal to noise volume. The unit used for volume variability may be decibel (dB). Pitch may be determined by detecting a frequency, that is, a highness/lowness of voice, during a speech, and calculating a mean of the detected frequency. The unit used for pitch may be hertz (Hz). Pitch variability may be determined calculating a standard deviation of the detected frequency. The unit used for pitch variability may be hertz (Hz). Fluidity may be determined by detecting a rate of micropauses per phonation time. Micropauses may be understood as less than 0.3 second pauses between spoken syllabi in continuous speech. Phonation time may be understood as a period during which sound is produced by a speaker. The unit used for fluidity is a percentage. Pace may be determined by detecting a number of words during speech and calculating a mean of the number of words over time. The unit used for pace may be words per minute (wpm). Pace variability may be determined by calculating a standard deviation of the number of words over time. The unit used for pace variability may be words per minute (wpm). Pauses may be determined by detecting a number of macropauses and a number of words during speech and calculating a number of macropauses per word. Macropauses may be understood as transition periods or long silences between words. The unit used for pauses may be pauses per word or a percentage. Facial expressions from neutral may be determined by detecting a number of times a speaker changes from a neutral expression to an emotional expression during a speech and calculating a means of the number of changes to emotional expressions over time. Facial expressions variability may be determined by detecting a number of times a speaker changes an emotional expression to a different emotional expression during a speech and calculating a mean of the number of changes over time. Filler words may be determined by detecting a number of filler sounds, that is, words or sounds that can be understood as meaningless sounds, and a total number of words during speech, and calculating a ratio of the filler sounds and the total number of words. Word complexity may be understood as a percentage of long words in speech, wherein words of at least three syllables may be defined as long. Word complexity of a speech may be determined, for example, by using Linsear Write readability metric. In the Linsear Write readability metric short words, that is, words containing at most two syllables, receive one point and long words receive three points. The received points are added up and the result is first divided by the number of sentences, and then if the result is greater than 20, divided by two, and if the result is smaller than or equal to 20, two is first subtracted from the result and it is then divided by two. Word complexity may be determined, for example, as an average of the Linsear Write metric value of the sentences in the speech. Openness, extraversion, emotional stability, agreeableness, and conscientiousness may be understood as personality traits of the speaker that may be assessed from a speech. Openness may be understood as a degree of intellectual curiosity, a desire to seek new experiences, and a preference of novelty and variety. Openness metric of a speech may be determined by, for example, analysing the words in the speech and using facial expressions and pitch. The range of openness metric values may extend from "very closed" to "very open", which may be expressed as a numerical value range such as 0-10 or 1-100, for example. Extraversion may be understood as a degree of assertiveness and sociability. Extraversion metric of a speech may be determined by, for example, analysing the words in the speech and using facial expressions and pace. The range of extraversion metric values may extend from "very introverted" to "very extroverted" , which may be expressed as a numerical value range such as 0-10 or 1-100, for example. Emotional stability may be understood as a degree of reacting to unpleasant emotions such as anger or anxiety. Emotional stability metric of a speech can be determined by, for example, analysing the words in the speech and using facial expressions, pitch, fluidity, and pauses. The range for emotional stability metric values may extend from "very sensitive" to "very balanced", which may be expressed as a numerical value range such as 0-10 or 1-100, for example. Agreeableness may be understood as a degree of compassion, cooperation, and friendliness. Agreeableness metric of a speech may be determined by, for example, analysing the words of the speech and using facial expressions. The range of agreeableness metric values may extend from "very disagreeable" to "very agreeable" , which may be expressed as a numerical value range such as 0-10 or 1-100, for example. Conscientiousness may be understood as a degree of self-discipline, planning, and dutifulness. Conscientiousness metric of a speech may be determined by, for example, analysing the words of the speech and using facial expressions and pace. The range for conscientiousness metric values may extend from "very easy-going" to "very conscientious", which may be expressed as a numerical value range such as 0-10 or 1-100, for example. The words of the speech may be analysed by using a machine learning model trained to perform psycholinguistic evaluations. Psycholinguistics may be understood as a science studying interrelations between language and psychological aspects (personality). A non-limiting example includes using a deep learning based model such as a neural network for predicting personality model traits such as The Big Five personality characteristics. The trained machine learning model 122 uses as input the determined technical parameters and outputs assessed hormone level impact labels. The machine learning model parameters the trained machine learning model 122 uses to obtain an assessment may be received from the machine learning training unit 112 of the training equipment 110. The assessed hormone level impact labels obtained as the output of the trained model 122 may be displayed, for example, on the display 123 with for example text, numbers, images, and/or indicators showing an increased hormone level, a decreased hormone level, or an unaltered hormone level for one or more hormones.

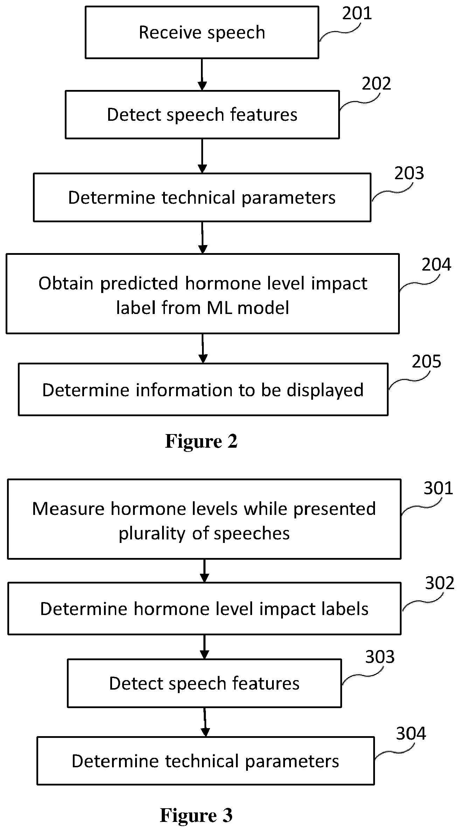

In the illustrated example of Figure 2 hormone level impact of a speech is predicted by an artificial intelligence model trained to map a plurality of technical parameters of a speech to at least one hormone level impact label.

Referring to Figure 2, a speech is received in block 201 from a user. The speech may be received, for example, by recording data such as, for example, audio, video, photogrammetric, and/or volumetric data with the assessing equipment or by transferring a previously recorded data to the assessing equipment. A plurality of speech features is detected in block 202 of the speech. The plurality of speech features may be such as explained previously with Figure 1. A plurality of technical parameters of the speech is determined in block 203 from the detected plurality of speech features. At least one predicted hormone level impact label is obtained in block 204 by inputting the plurality of technical parameters determined to a machine learning model trained to map the plurality of technical parameters to at least one hormone level impact label, the trained machine learning model outputting the at least one predicted hormone level impact label. Information to be displayed to the user is determined in block 205 using the at least one predicted hormone level impact label obtained. The information may be displayed, for example, as text, numbers, images, and/or indicators indicating or visualising how the impact is located on a range and/or showing an increased hormone level, a decreased hormone level, or an unaltered hormone level for one or more hormones.

It should be noted that a sufficient number of technical parameters to obtain an assessment of at least one predicted hormone level impact label may be determined by the trained machine learning model. The sufficient number technical parameters may not be met, for example, if an audio and/or video recording is too short, or if the quality of data is not sufficient. If the sufficient number of determined technical parameters is not fulfilled in block 203, the application may prompt the user to record or transfer new or additional data.

In the illustrated example of Figure 3, data for training a machine learning model is collected by measuring human hormone level changes and determining technical speech parameters.

Referring to Figure 3, at least one hormone level is measured in block 301 a plurality of times from a set of individuals while being presented a plurality of speeches, per an individual, one speech at a time. The at least one hormone level is measured the plurality of times during one speech to obtain information on how the at least one hormone level changes while the individual is being presented the said speech of the plurality of speeches. The measuring of hormone levels may be performed as explained in more detail with Figure 1. The hormone levels measured are tied to a corresponding individual of the set of individuals and to a corresponding speech of the plurality of speeches. At least one hormone level impact label is determined in block 302, per a speech, using the at least one hormone level measured from the set of individuals the plurality of times. The at least one hormone level impact label may be determined, for example, as an average of the changes in hormone levels measured. The at least one hormone level impact label is determined for training a machine learning model. Hormone level impact labels determined are tied to a corresponding speech of the plurality of speeches. At least one speech feature of a plurality of speech features is detected in block 303, per a speech of the plurality of speeches. Speech features detected are tied to a corresponding speech of the plurality of speeches. At least one technical parameter value of a plurality of technical parameters is determined in block 304, per a speech of the plurality of speeches from the at least one speech feature detected. The at least one hormone level impact label and the at least one technical parameter value may be stored in a database, as explained in more detail with Figure 1.

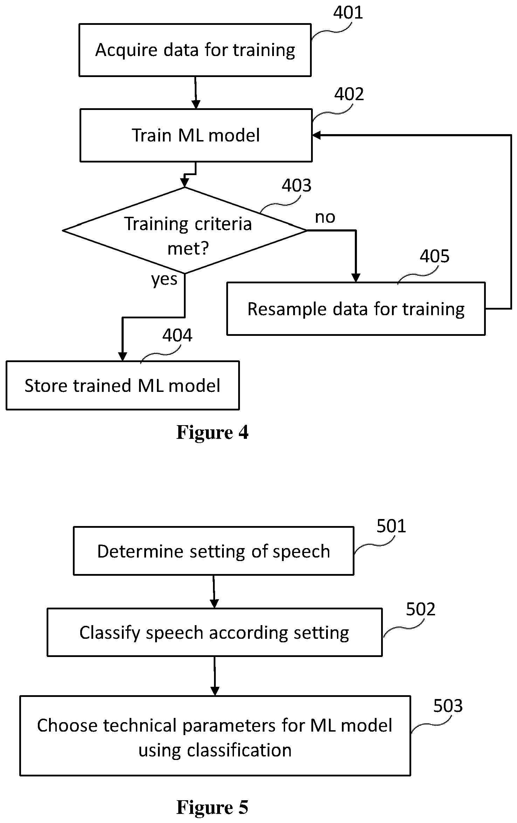

In the illustrated example of Figure 4, a machine learning model is trained with technical parameters data and hormone level impact data.

Data for training a machine learning model is acquired in block 401. The data may be acquired from a database stored as explained in more detail with Figure 3. The machine learning model is trained in block 402 using at least one technical parameter of a plurality of technical parameters, per a speech, as input and at least one hormone level impact label, per a speech, as a target output. The machine learning model may be any machine learning model suitable for supervised learning, for example, a linear regression model. Alternatively, other machine learning models may be used, such as for example a non-linear transformation model, a logistic regression model, a generalized additive model, a neural network, or a regression tree. The linear regression model may be understood as a machine learning algorithm that is trained to find a linear relationship between input data and target outputs. It is checked in block 403 if criteria for sufficient training are met by the trained machine learning model. The trained machine learning model may be evaluated by, for example, k-fold cross-validation, where a limited sample of the training data is not used for training the machine learning model but for evaluating the quality of the trained machine learning model. The criteria for sufficient training may be determined, for example, by a percentage of hormone impact labels of the evaluation data being assessed correctly by the trained machine learning model. If the criteria for sufficient training are met (block 403: yes), the trained machine learning model parameters may be stored in block 404 for assessing hormone level impact of speech in the assessment equipment, as explained in more detail with Figure 2. If the criteria for sufficient training are not met (block 403: no), the process continues in block 405 by resampling the data for training the machine learning model and then (re)training in block 402 the machine learning model with the resampled data.

In the illustrated example of Figure 5, classification of speech according to a setting is utilised for choosing appropriate technical parameters to be used as an input for training a machine learning model and consequently in using the trained machine learning model to assess hormone level impact of a speech.

Referring to Figure 5, a setting where a speech is held is determined in block 501. The setting where the speech is held may be, for example, real world, metaverse, social media, and/or a combination of a plurality of settings. Also, a more detailed determination may be performed, such as for example a conference, an onsite meeting or an online meeting, or a recorded or live video published in social media. The speech is classified in block 502 according to the setting determined. Technical parameters for machine learning model input are chosen in block 503 using the classification. The technical parameters chosen may comprise technical parameters appropriate in view of the setting where the speech is held. Also, additional parameters may be used depending on the setting, such as for example size, light, and/or acoustics of a real world or metaverse venue.

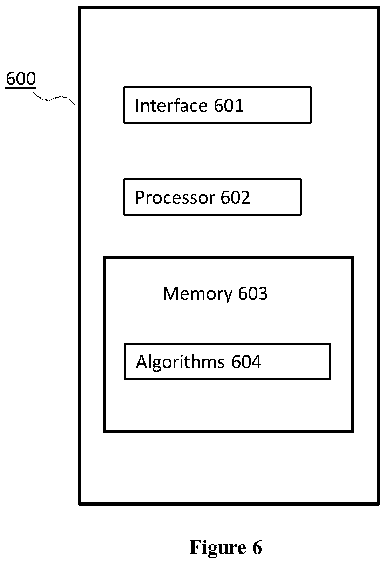

Figure 6 is a simplified block diagram illustrating some units for an apparatus (device, equipment) 600 configured to perform at least some functionality described above, for example by means of Figures 1 to 5 and any combination thereof. In the illustrated example, the apparatus 600 comprises one or more interface (IF) entities 601, such as one or more user interfaces, and one or more processing entities 602 connected to the various interface entities 601 and to one or more memories 603.

The one or more interface entities 601 are entities for receiving and transmitting information, such as communication interfaces comprising hardware and/or software for realizing communication connectivity according to one or more communication protocols, or for realizing data storing and fetching.

A processing entity 602 is capable to perform calculations and configured to implement at least part of the functionalities/operations described above, for example by means of any of Figures 1 to 5 and any combination thereof, with corresponding algorithms 604 stored in the memory 603. The entity 602 may include one or more processors, controllers, control units, micro-controllers, etc., configurable to carry out embodiments, examples, implementations, or operations described above, for example by means of any of Figures 1 to 5 and any combination thereof. Generally, a processor is a central processing unit, but the processor entity 602 may be an additional operation processor or a multicore processor or a microprocessor.

A memory 603 is usable for storing a computer program code required for one or more or the functionalities/operations described above, for example by means of any of Figures 1 to 5 and any combination thereof, that is, the algorithms 604 for implementing the functionalities/operations described above by means of any of Figures 1 to 5 and any combination thereof. The memory 603 may also be usable for storing, at least temporarily, other possible information required for one or more of the +functionalities/operations described above, for example by means of any of Figures 1 to 5 and any combination thereof. The memory may comprise a data buffer that may, at least temporarily, store speech data, technical parameters, hormone level impact labels, and information to be displayed.

As a summary, the methods described herein, for example by means of any of Figures 1 to 5 and any combination thereof, may be configured as a computer or a processor, or a microprocessor, such as a single-chip computer element, or as a chipset, or one or more logic gates including at least a memory for providing storage area used for arithmetic operation and an operation processor for executing the arithmetic operation. Each or some or one of the algorithms for functionalities/operations described above, for example by means of any of Figures 1 to 5 and any combination thereof, may be comprised in one or more computer processors, application-specific integrated circuits (ASIC), digital signal processors (DSP), digital signal processing devices (DSPD), programmable logic devices (PLD), field-programmable gate arrays (FPGA), graphics processing units (GPU), and/or other hardware components that have been programmed and/or will be programmed by downloading computer program code (one or more algorithms) in such a way as to carry out one or more functions of one or more embodiments/ examples.

An embodiment provides a computer program embodied on any client-readable distribution/data storage medium or memory unit(s) or article(s) of manufacture, comprising program instructions executable by one or more processors/computers, which instructions, when loaded into an apparatus (device, equipment), constitute an entity providing corresponding functionality, or at least part of the corresponding functionality. Programs, also called program products, including software routines, program snippets constituting "program libraries", applets and macros, can be stored in any medium, including non-transitory computer readable storage medium, and may be downloaded into an apparatus. In other words, each or some or one of the algorithms for one or more functions/operations described above, for example by means of any of Figures 1 to 5 and any combination thereof, may be comprised in an element that comprises one or more arithmetic logic units, a number of special registers, and control circuits.

It will be obvious to a person skilled in the art that, as the technology advances, the inventive concept can be implemented in various ways. The invention and its embodiments are not limited to the examples described above but may vary within the scope of the claims.

## Claims

1. A computer-implemented method comprising:
receiving a speech from a user;
detecting, from the speech, at least one of a plurality of speech features;
determining at least one parameter value of a plurality of technical parameters using the at least one speech feature detected;
inputting the at least one technical parameter value determined to a machine learning model trained to map the plurality of technical parameters to at least one hormone level impact label;
obtaining at least one predicted hormone level impact label of the speech from the machine learning model; and
determining information related to the at least one predicted hormone level impact label obtained to be displayed to the user.

2. A computer-implemented method comprising:
acquiring data for training a machine learning model, wherein the data comprises a plurality of hormone level impact labels determined from a plurality of hormone levels measured, per an individual per a speech, from a set of individuals while being presented a plurality of speeches, and parameter values for a plurality of technical parameters determined from a plurality speech features detected from the plurality of speeches;
training the machine learning model to map the plurality of technical parameters to at least one hormone level impact label of the plurality of hormone level impact labels;
storing, when criteria for sufficient training are met, the trained machine learning model.

3. A computer-implemented method according to claim 2, wherein the plurality of hormone levels comprises a level of cortisol, a level of dopamine, a level of oxytocin, a level of dehydroepiandrosterone, and/or a level of serotonin.

4. A computer-implemented method according to any of the preceding claims, wherein the machine learning model is a regression model.

5. A computer-implemented method according to any of the preceding claims, wherein the plurality of technical parameters comprises at least one of: perceived volume, volume variability, pitch, pitch variability, fluidity, pace, pace variability, pauses, facial expressions from neutral, facial expressions variability, filler words, word complexity, openness, extraversion, emotional stability, agreeableness, and/or conscientiousness.

6. A computer-implemented method according to any of the preceding claims, further comprising:
classifying the plurality of speeches according to a setting where a speech is held, wherein the setting comprises at least one of real world, metaverse, and/or social media; and
choosing the plurality of technical parameters to be determined for the machine learning model using the classification.

7. An apparatus comprising means for performing a method according to any of the preceding claims.

8. A computer-readable medium comprising program instructions which, when run by an apparatus, cause the apparatus to carry out at least a method according to any of claims 1-6.

9. A computer-readable medium of claim 8, wherein the computer-readable medium is a non-transitory computer-readable medium.
